(19) Europäisches Patentamt
European Patent Office
Office européen des brevets

(11) EP 1 178 010 B1

(12) **EUROPEAN PATENT SPECIFICATION**

(45) Date of publication and mention of the grant of the patent:
**30.03.2005 Bulletin 2005/13**

(51) Int Cl.⁷: **C01C 1/20**, C01C 3/20

(21) Application number: **01204073.9**

(22) Date of filing: **07.01.1998**

(54) **Colorless aqueous ammonium sulfide solution**

Farblose wässrige Ammoniumsulfidlösung

Solution aqueuse de sulfide d'ammonium incolore

(84) Designated Contracting States:
**BE DE FR GB**

(30) Priority: **10.01.1997 US 34727 P**

(43) Date of publication of application:
**06.02.2002 Bulletin 2002/06**

(62) Document number(s) of the earlier application(s) in accordance with Art. 76 EPC:
**98903464.0 / 0 951 485**

(73) Proprietors:
- **E.I. DU PONT DE NEMOURS AND COMPANY Wilmington, Delaware 19898 (US)**
- **COMMONWEALTH SCIENTIFIC AND INDUSTRIAL RESEARCH ORGANISATION Clayton, VIC 3168 (AU)**

(72) Inventors:
- **Anderson, Albert Gordon Wilmington, Dealware 19808 (US)**
- **Gridnev, Alexei Wilmington, Delaware 19803 (US)**

(74) Representative: **Abitz, Walter, Dr.-Ing.
Patentanwälte Abitz & Partner
Postfach 86 01 09
81628 München (DE)**

(56) References cited:
**US-A- 5 057 612**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 178 010 B1

## Description

This invention concerns the preparation of imidazoline nitroxides (1) as further defined hereafter:

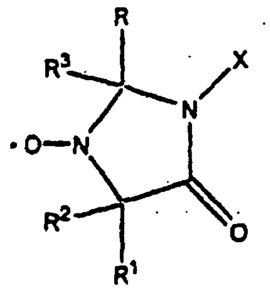

Formula (1)

wherein:

R, $R^1$, $R^2$, $R^3$ are each independently selected from the group consisting of $C_1$ to $C_{18}$, alkyl, substituted $C_1$ to $C_{18}$ alkyl, $C_6$ to $C_{18}$ aryl, $C_6$ to $C_{18}$ substituted aryl; R groups that are in a geminal position with respect to each other can together form a 4-8 membered ring; R groups that are in a cis position with respect to each other can together form a 4-8 membered ring; and

X is selected from the group consisting of hydrogen, $C_1$ to $C_{18}$ alkyl, substituted $C_1$ to $C_{18}$ alkyl, $C_6$ to $C_{18}$ aryl, $C_6$ to $C_{18}$ substituted aryl; acyl; X and R can form a 5-8 membered ring; X and $R^3$ can form a 5-8 membered ring

The invention further concerns a colorless aqueous, ammonium sulfide solution containing sodium thiocyanate and substantially no ammonium polysulfide.

Use of the nitroxides of Formula 1 (or the corresponding alkoxyamines) offers signiftcant advantages over nitroxides previously employed in nitroxide-mediated poiymeritation: homopolymers, statistical copolymers and block copolymers which have controlled molecular weight, a narrow molecutar weight distribution and a defined end-group fimcdonwity can be synthesized. The method is also adaptable to the preparation. of multi-block and graft and other polymers of more complex architecture. With appropriate selection of the substituents, R, $R^1$, $R^2$, $R^3$, and X, (defined hereafter), the use of the nitroxides (1) offers lower polydispersities and better living character than, for example, TEMPO and derivatives.

Further advantages are that (a) the nitroxides (1) and the derived alkoxyamine are synthesized from readily available precursors, by a simple experimental route; (b) they are subject to fewer side reactions (e.g., disproportionation of propagating radical with nitroxide or chain transfer to nitroxide); and (c) they are involatile. This provides an advantage over many of the most commonly used nitroxides such as TEMPO and many of its derivatives, and di-t-butyl nitroxide, which are odorous.

The process can be run continuously or in batch and can be carried out as a solution, emulsion, suspension or bulk polymerization using procedures well known in the art.

This invention concerns particular the preparation of nitroxides of the Formula (1) useful in the polymerization process wherein:

R, $R^1$, $R^2$, $R^3$ are each independently selected from the group consisting of $C_1$ to $C_{18}$ alkyl, substituted $C_1$ to $C_{18}$ alkyl, $C_6$ to $C_{18}$ aryl. $C_6$ to $C_{18}$ substituted aryl; R groups in a geminal position with respect to each other can together form a 4-8 membered ring; and R groups in a cis position with respect to each other can together form a 4-8 membered ring; and

X is selected from the group consisting of $C_1$ to $C_{18}$ alkyl, substituted $C_1$ to $C_{18}$ alkyl, $C_6$ to $C_{18}$ aryl, $C_6$ to $C_{18}$ substituted aryl; acyl; X and R can form a 5-8 membered ring; and X and $R^3$ can form a 5-8 membered ring;

with the proviso that R, $R^1$, $R^2$, $R^3$ and X are not all, methyl.

Preferred nitroxides selected from the group above are the following:

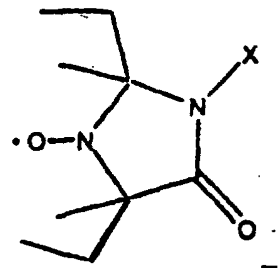

Formula (4)

where X is selected from the group consisting of alkyl, optionally substituted alkyl, benzyl; and

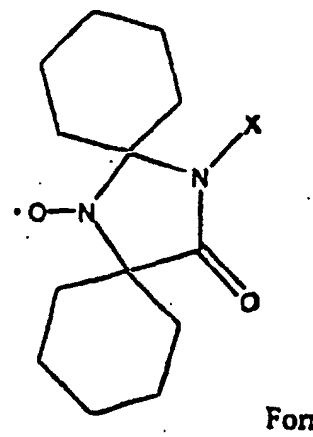

Formula (5)

where X is alkyl of $C_1$ to $C_{18}$.

This invention also includes a process for making the nitroxides of Formula (1). The process comprises reacting an aminonitrile and a ketone to form a cyanoimine, and reacting said imine with hydrogen sulfide to produce a linear thioamide, and cyclizing said linear thioamide to form a 2,2,5,5,-tetrasubstituted-imidazolidin-4-thione, and converting the cyclic thioamide to the corresponding cyclic amide and then converting the final imidazolidine-4-one to the nitroxide.

In particular, the process for making nitroxides of Formula (1) involves: (i) preparing a colorless aqueous ammonium sulfide solution containing sodium thiocyanate by titrating an aqueous ammonium sulfide solution containing ammonium polysulfide with sodium cyanide under nitrogen; (ii) sequentially adding an aminonitrile and a ketone to the aqueous ammonium sulfide solution under nitrogen; (iii) adding base and then neutralizing; and (iv) oxidizing the reaction product of step (iii) to form the nitroxide.

Alternatively, in process step (ii) aminonitrile can be replaced by a mixture of ketone, ammonium chloride, and sodium or potassium cyanide. In another embodiment of this process, the process is stopped before addition of sodium tungstate and the corresponding cyclic amine/amide is isolated. Process step (ii) can be performed at a temperature of between 20° and 80°C, preferably between 30° and 60°C, and most preferably at 54°C. The base is preferably sodium carbonate or sodium hydroxide, most preferably sodium hydroxide. Any convenient acid can be used for the neutralization, the preferred acid is sulfuric acid. In this process, the concentration of hydrogen peroxide is preferably 20 to 50%, most preferably 30%. The preferred oxidants for the amine to nitroxide transformation are $H_2O_2$/tungstate, dimethyldioxirane, $H_2O_2$/acetic acid.

The most commonly used nitroxides in nitroxide-mediated living free-radical polymerizations have been 2,2,6,6-tetramethylpiperidin-N-oxyl (TEMPO) and derivatives of this compound and di-t-butyl nitroxide (diBuNO).

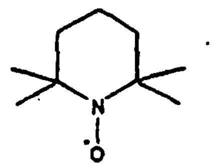

TEMPO

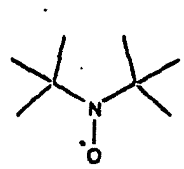

diBuNO

These and other nitroxides/alkoxyamines that are conventionally used in nitroxide-mediated living free-radical polymerizations are inherently of high cost. Substantial cost improvements for the overall process can therefore be achieved by the use of nitroxide (1), a material obtainable from inexpensive precursor by a simple experimental route.

It has been found, that in various polymerizations, the use of certain 2,2,5,5-tetraalkylimidazolin-4-one-1-oxyl derivatives in nitroxide-mediated polymerization offer lower polydispersities for polymers than is obtained with other nitroxides used for this purpose (e.g., TEMPO and derivatives, or diBuNO).

In the context of the present invention, low polydispersity polymers are those with polydispersities that are significantly less than those produced by conventional frec radical polymerization. In conventional free radical polymerization, polydispersities (the polydispersity is defined as the ratio of the weight average and number average molecular weights - $\overline{M}_w/\overline{M}_n$) of the polymers formed are typically in the range 1.6-2.0 for low conversions (<10%) and can be substantially greater than this for higher conversions. Polydispersities obtained with the present invention are usually less than 1.5, often less than 1.3 and, with appropriate choice of the nitroxides (1) / alkoxyamines and the reaction conditions, can be less than 1.1. The low polydispersity can be maintained at high conversions.

Polydispersities in nitroxide-mediated polymerization are believed to depend on a number of factors. These include (i) the rate of exchange between active and dormant species which is largely determined by the rate of bond homolysis between N-O and the adjacent moiety for the alkoxyamines involved either as initiator species or formed during the polymerization (for a discussion on this subject see Moad and Rizzardo, *Macromolecules* 1995, *28*, 8722-8); and (ii) the significance of various side reactions.

For polymerizations involving nitroxides (1) the rate of bond homolysis between N-O and the adjacent moiety and polydispersities obtained depend on the particular nitroxide or alkoxyamine used and in particular on the substituents R, $R^1$, $R^2$, $R^3$ and X. A preferred group of nitroxides in this context are the N-alkyl-2,2,5,5-tetraalkylimidazolin-4-one-1-oxyl compounds (i.e., (1) X=alkyl, for example, 2,5-bis(spirocyclohexyl)-3-methylimidazolidin4-one-1-oxyl (NO-88-Me)) which are seen to offer the lowest polydispersities in styrene polymerizations or copolymerizations. Also preferred within each class (X=alkyl and X=H) are those (1) with more bulky R-$R^3$.

The following are structures of nitroxides described herein:

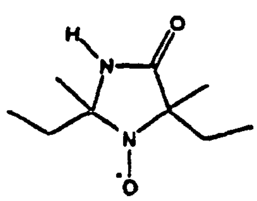

NO-67

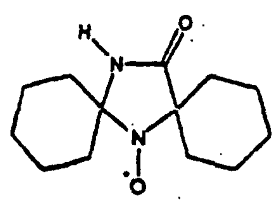

NO-88

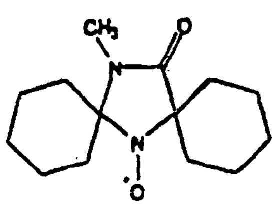

NO-88-Me

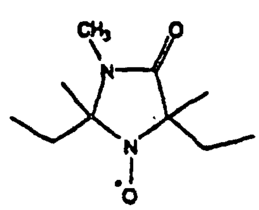

NO-67-Me

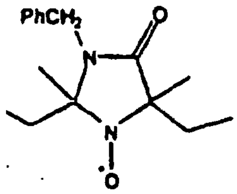 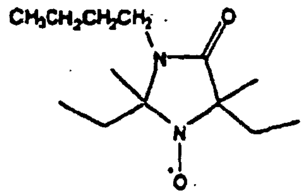

NO-67-Bn NO-67-nBu

It is believed that an important side reaction in nitroxide-mediated polymerization is disproportionation between the nitroxide and the propagating species. It has been found that in methyl methacrylate (MMA) polymerization the use of 2,2,5,5-tetiaalkylimidazolin-4-one-1-oxyl derivatives offer low polydispersities and good living character for polymerizations.

In the synthesis of nitroxides of Formula (1), the product nitroxide can be isolated by conventional means, preferably from the reaction mixture by filtration or by extraction with an organic solvent that is substantially insoluble in water.

It has been found that the ammonium polysulfide reacts with either aminonitnie or cyanide ion, thereby reducing the amount of cyanide below stoichiometric proportions thus lowering the overall yield. This can be prevented by prior addition of cyanide ion to the point of decolorization of the polysulfide and formation of harmless thiocyanate.

The procedure disclosed herein for synthesis of nitroxides (1, X=H) is as follows:

**SCHEME 1**

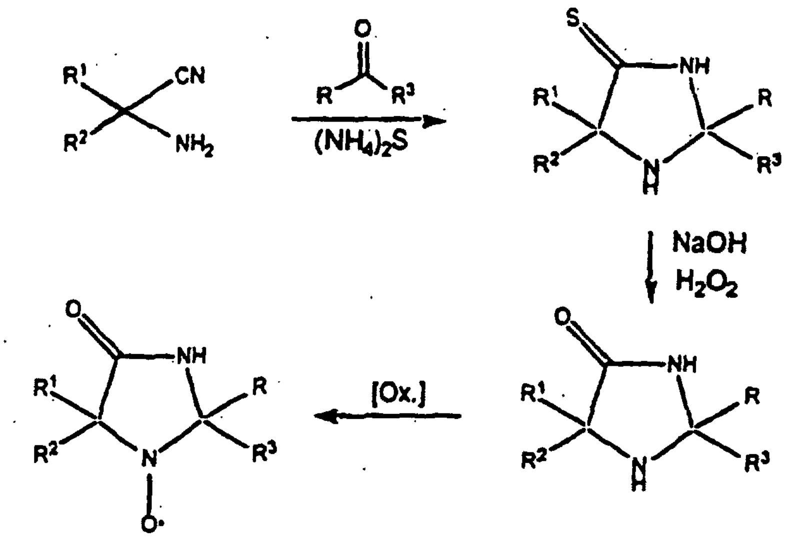


(1) (6)

EXAMPLES

General Experimental Conditions

Monomers were purified (to remove inhibitors) and flash distilled immediately prior to use. Degassing was accomplished by repeated freeze-evacuate-thaw cycles. Once degassing was complete ampoules were flame sealed under vacuum and completely submerged in an oil bath at the specified temperature for the specified times. The percentage conversions were calculated gravimetricalty.

NMR spectra were obtained on a Bruker (200 MHz) spectrometer and $CDCl_3$ was used as solvent.

**Examples 6-11**

**MMA Polymerizations**

The following section reports results of methyl methacrylate polymerizations in the presence of the azo-initiator, 2,2'-Azobis(2,4-dimethylvaleronitrile) (Vazo®-52) and different nitroxides. The results for NO-67 (Examples 6-9) and NO-88 (Examples 10-12) are shown in Tables 2 and 3 respectively. The effectiveness of these nitroxides is compared with other nitroxides in Table 4. Further sampling after one hour of reaction time shows little or no increase in molecular weight or conversion. The five-membered ring nitroxides (NO-67 and NO-88) gave the most favorable results (narrowest polydispersity). In all cases, the product is believed to be a MMA macromonomer formed by loss of a hydrogen atom from the propagating species to the nitroxide (i.e., reaction by disproportionation rather than combination).

**Procedure**:

A stock solution was prepared containing MMA (10 ml, 9.36 g) Vazo®-52 (13.43 mg, 0.054 mmol), and NO-67 (14.2 mg, 0.077 mmol). 3 ml of the stock solution was transferred to each of three ampoules which were then degassed through 3 freeze-thaw cycles, sealed, and heated at 90 °C for the indicated times.

Table 2:

| Bulk MMA Polymerization with Vazo®-52 and NO-67, 90°C. | | | | | |
|---|---|---|---|---|---|
| Example | time/h | $\bar{M}_n$ | $\bar{M}_w/\bar{M}_n$ | % Conv.[a] | calc. $\bar{M}_n$ |
| 6 | 0.5 | 31737 | 1.68 | 24.9 | 30305 |
| 7 | 1 | 38021 | 1.47 | 25.3 | 30730 |
| 8 | 6 | 35709 | 1.57 | 38.2 | 46411 |

[a] % conversion evaluated from mass of polymer obtained.

Procedure:

A stock solution was prepared containing MMA (9 ml, 8.42 g) Vazo®-52 (12.08 mg, 0.049 mmol), and NO-88 (17.16 mg, 0.069 mmol). 3 ml of the stock solution was transferred to each of three ampoules which were then degassed through 3 freeze-thaw cycles, sealed, and heated at 90 °C for the indicated times.

Table 3:

| Bulk MMA Polymerization with Vazo®-52 and NO-88, 90°C. | | | | | |
|---|---|---|---|---|---|
| Example | time/h | $\bar{M}_n$ | $\bar{M}_w/\bar{M}_n$ | % Conv.[a] | calc. $\bar{M}_n$ |
| 9 | 0.5 | 308 | 1.03 | 0.7 | 866 |
| 10 | 1 | 6472 | 1.44 | 6.4 | 7822 |
| 11 | 6 | 7890 | 1.44 | 5.4 | 6590 |

[a] % conversion evaluated from mass of polymer obtained.

## TABLE 4

**Table 4: MMA Polymerizations after 1 hour at 90°C with VAZO®-52 and Nitroxide[i]**

| Nitroxide | $\bar{M}_n$ | %Conv. | Calculated $\bar{M}_n$ | $\bar{M}_w/\bar{M}_n$ |
|---|---|---|---|---|
| 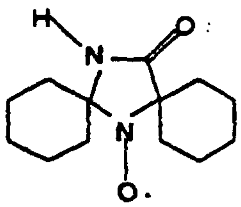 NO-88 | 6472 | 6.4 | 7822 | 1.44 |
| 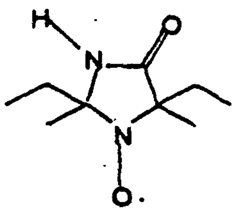 NO-67 | 38021 | 24.9 | 30730 | 1.47 |
| | | | | |
| 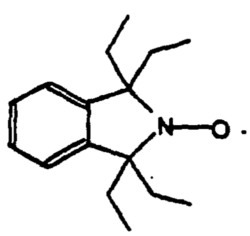 | 18294 | 18.4 | 21528 | 1.71 |

[i] Reaction conditions similar to those used for experiments described in Tables 2 and 3.

| | | | | |
|---|---|---|---|---|
| TEISO | | | | |
| 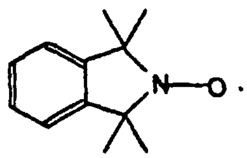 | 10515 | 11.2 | 13554 | 2.24 |
| 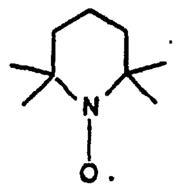 TEMPO | 22073 | 14.3 | 17322 | 3.11 |
| 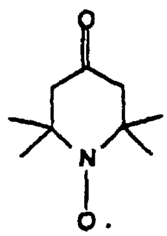 | 16959 | 17.5 | 21181 | 3.30 |
| 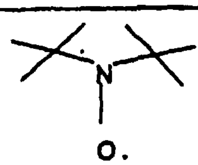 DiBuNO | 41904 | 15.4 | 18696 | 3.15 |
| 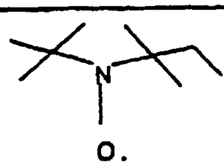 | 19191 | 17.9 | 21735 | 4.10 |

VAZO® is a registered trademark of E. I. du Pont de Nemours and Company. The particular VAZO® compositions referred to herein comprise the following compounds:

VAZO®52 2,2'-azobis(2,4-dimethylvaleronitrile),
VAZO®64 2,2'-azobisisobutyronitrile,
VAZO®67 2,2'-azobis(2-methylbutyronitrile), and
VAZO®88 1,1'-azobis(cyanocyclohexane),

**<u>Examples 12 to 27</u>**

**Styrene polymerization**

A series of styrene polymerizations was conducted in the presence of NO-67, NO-88, and the N-substituted imidazolidinone nitroxides, NO-67-Me, NO-88-Me, NO-67-Bn and NO-67-nBu) and benzoyl peroxide initiator. The polymerization was carried out at 130°C for a period of times indicated in Table 5 below. Results are summarized in Table 5.

Procedure:

The following six solutions were prepared.

(i) Styrene (5 ml), NO-88 (72.75 mg) and benzoyl peroxide (35.35 mg).
(ii) Styrene (10 ml), NO-88-Me (154.00 mg) and benzoyl peroxide (70.70 mg).
(iii) Styrene (5 ml), NO-67 (56.75 mg) and benzoyl peroxide (35.35 mg).
(iv) Styrene (10 ml), NO-67-Me (122.00 mg) and benzoyl peroxide (70.70 mg).
(v) Styrene (5 ml), NO-67-Bn (84.32 mg) and benzoyl peroxide (35.35 mg).
(vi) Styrene (5 ml), NO-67-nBu (73.88 mg) and benzoyl peroxide (35.35 mg). Aliquots (2 ml) of these solutions were transferred into ampoules and the contents were degassed by three freeze-thaw cycles. The ampoules were then sealed and heated at 130°C for times indicated in Table 5. The ampoules were cooled, opened and the reaction mixture reduced *in vacuo* to a residue which was dried to constant weight and analyzed by GPC.

Table 5:

| GPC molecular weight data of polystyrene prepared via polymerizations of styrene with nitroxides and benzoyl peroxide at 130 °C | | | | | |
|---|---|---|---|---|---|
| Example | Nitroxides | Time/hr | $\overline{M}_n$ | $\overline{M}_w/\overline{M}_n$ | % Conv. |
| 12 | NO-88 | 23 | 16047 | 1.23 | 99.0 |
| 13 | NO-88-Me | 2 | 780 | 1.18 | 5.9 |
| 14 | NO-88-Me | 4 | 3115 | 1.13 | 20.9 |
| 15 | NO-88-Me | 8 | 8765 | 1.09 | 56.0 |
| 16 | NO-88-Me | 18 | 16271 | 1.09 | 96.0 |
| 17 | NO-88-Me | 23 | 16300 | 1.09 | 99.0 |
| 18 | NO-67 | 23 | 16043 | 1.49 | 99.0 |
| 19 | NO-67-Me | 2 | 502 | 1.36 | 5.0 |
| 20 | NO-67-Me | 4 | 1380 | 1.29 | 9.9 |
| 21 | NO-67-Me | 8 | 2499 | 1.34 | 22.4 |
| 22 | NO-67-Me | 18 | 4693 | 1.29 | 49.4 |
| 23 | NO-67-Me | 23 | 8075 | 1.24 | 60.0 |
| 24 | NO-67-Bn | 4 | 1402 | 1.23 | 9.1 |
| 25 | NO-67-Bn | 18 | 5102 | 1.28 | 47.4 |
| 26 | NO-67-nBu | 4 | 1430 | 1.22 | 8.8 |
| 27 | NO-67-nBu | 18 | 6013 | 1.25 | 53.0 |

The proton-NMR spectrum of a polystyrene sample ($\overline{M}_n$ 3115) of Example 14 had signal at $\delta$ 2.90 ppm clearly indicating the presence of the N-methyl of the 2.5-bis(spirocyclohexyl)-3-methylimidazolidin-4-one-1-oxyl (NO-88-Me) end group.

**Examples 28-31**

**Acrylate Polymerization**

Polymerization of *tert*-butyl acrylate was carried out in sealed tubes at 120°C using the alkoxyamine, 1-(2-*tert*-butoxy-1-phenylethoxy)-2,5-bis(spirocyclohexyl)-3-methylimidazolidin-4-one as initiator-terminator. This example demonstrates the acrylate polymers with low polydispersity (1.3-1.4) can be obtained.
Two set of experiments were conducted:

(i) A stock solution of the alkoxyamine (71.3 mg), tert-butyl acrylate (1.0 ml) in benzene (4.0 ml) was prepared. Aliquots (2.0 ml) were transferred into ampoules (x2) and the contents were degassed by three freeze-thaw cycles.

The ampoules were then sealed and heated at 120°C for 24 hours and 49 hours respectively. Results are shown in Table 6 below.

Table 6:

| *tert* - Butyl acrylate polymerizations in the presence of alkoxyamine in benzene at 120°C | | | | |
|---|---|---|---|---|
| Example | Time (hr) | $\bar{M}_n$ | $\bar{M}_w/\bar{M}_n$ | % Conv. |
| 28 | 24 | 1525 | 1.39 | 28.0 |
| 29 | 49 | 1830 | 1.32 | 34.4 |

The proton-NMR spectrum of a sample of poly(tert-butyl acrylate) of Example 28 ($\bar{M}_n$ 1525) had signals at δ 7.10 ppm indicating the presence of phenyl group (cf. δ 7.30 ppm for the original alkoxyamine used) and d 2.90 ppm indicating the presence of the N-methyl group of the NO-88-Me.

(ii) A stock solution of alkoxyamine (71.3 mg), tert-butyl acrylate (5.0 ml) was prepared. Aliquots (2.0 ml) were transferred into ampoules (x2) and the contents were degassed by three freeze-thaw cycles. The ampoules were then sealed and heated at 120°C for 24 hours and 49 hours respectively. Results are shown in Table 7 below.

Table 7:

| Bulk*tert*-Butyl acrylate polymerizations in the presence of alkoxyamine at 120°C | | | | |
|---|---|---|---|---|
| Example | Time (hr) | $\bar{M}_n$ | $\bar{M}_w/\bar{M}_n$ | % Conv. |
| 30 | 24 | 8272 | 1.51 | 28.6 |
| 31 | 49 | 9005 | 1.41 | 39.8 |

**Examples 32 - 33**

**Block Copolymer Syntheses**

The following two examples [polystyrene-*block*-poly(4-methylstyrene) and polystyrene-*block*-poly(n-butyl acrylate)] demonstrate the synthesis of block copolymers. The samples were prepared by heating a narrow polydispersity polystyrene (derived from NO-88-Me) ($\bar{M}_n$, 8765, $\bar{M}_w/\bar{M}_n$ 1.09; see Table 5, Example 15) with 4-methylstyrene and n-butyl acrylate respectively. Results are excellent in both cases and give low polydispersity block copolymers.

**Example 32**

**Polystyrene-*block*-poly(4-methylstyrene)**

To an ampoule, a sample of polystyrene (250 mg) ($\bar{M}_n$ 8765, $\bar{M}_w/\bar{M}_n$ 1.09; Example 15) was dissolved in 1 ml of 4-methylstyrene (freshly distilled). The contents of the ampoule was degassed and sealed under vacuum. Subsequently, the mixture was polymerized at 130°C for 18 hours and gave a narrow polydispersity polystyrene-*block*-poly(4-methylstyrene) (0.85g, 95% conversion), $\bar{M}_n$ 36872, $\bar{M}_w/\bar{M}_n$ 1.14.

**Example 33**

**Polystyrene-*block*-poly(n-butyl acrylate)**

To an ampoule, a sample of polystyrene (250 mg) ($\bar{M}_n$ 8765, $\bar{M}_w/\bar{M}_n$ 1.09; Example 15) was dissolved in 1 ml of n-butyl acrylate (freshly distilled). The contents of the ampoule was degassed and sealed under vacuum. Subsequently, the mixture was polymerized at 130°C for 18 hours and gave a narrow polydispersity polystyrene-*block*-poly(n-butyl acrylate) (0.608g, 68% conversion), $\bar{M}_n$ 21526, $\bar{M}_w/\bar{M}_n$ 1.29.

**Examples 34-36**

**Statistical Copolymer Syntheses**

A series of styrene/acrylonitrile (62:38 molar ratio; the azeotropic composition) copolymerizations in the presence of N-substituted imidazolidinone nitroxides NO-88-Me, NO-67-Me and NO-67-Bn. The experiments were conducted thermally at 130 °C for 18 hours. Results are summarized in Table 8.

**Procedure**:

A stock solution (I) of freshly distilled styrene (7.27g) and acrylonitrile (2.27g) was prepared. Each ampoule contains stock solution (2g) and nitroxide ($1.23 \times 10^{-4}$ mol). The content was degassed, sealed and heated at 130°C for 18 hours.

**Examples 1-6**

**Syntheses of Nitroxides of Formula (1)**

The following Examples illustrate the novel process for synthesis of nitroxides (1, where X=H).

**Examples 1**

**Preparation of 2,5-diethyl-2,5-dimethylimidazolidin-4-one-1-oxyl (NO-67)** Preparation of 2,5-diethyl-2,5-dimethylimidazolidin-4-thione

A l liter 4-necked round-bottomed flask equipped with a mechanical stirrer, thermocouple thermometer, nitrogen bubbler, bleach-filled scrubber, and reflux condenser was charged with 17.4 g ammonium chloride (0.32 mol), 35.9 g AN-67 (0.3 mol, 87% 2-amino-2-methylpropionitrile in water, $^1$H NMR (ppm) of freshly distilled AN-67 in $D_2O$: 1.03 (t, 3H), 2.45 (s, 3H), 1.75 (q, 2H), 23.1 g 2-butanone (0.32 mol), and 132.9 g of 20% ammonium sulfide solution (0.39 mol). As the solution was heated to 50°C, a slight exotherm occurred that increased the temperature to 65°C and some ammonium hydrosulfide sublimed into the condenser. After 20 minutes, the temperature declined to 55°C and was held there for 18 hours. An oily liquid layer forms on top of the aqueous solution during the first 5 minutes. The yellow polysulfide color is discharged in the first minute; this is caused by reaction of cyanide ion with sulfur to give colorless thiocyanate ion and a decrease in yield. The next day, the solution was cooled to -15°C, 25 g of NaCl added to salt out the thione, and filtered cold to give 39 g of product. The mother liquor was treated with 10 g of $K_2CO_3$ to precipitate an additional 5 g of product. $K_2CO_3$ is more effective at salting out thiones, amides, and nitroxides than NaCl. The solids were combined to give 44 g (80% yield) of thione after 2 days of air drying, mp. 58-64°C. IR (nujol) 1540 cm-1; $^1$H NMR ppm ($D_2O$) 0.96 (overlapping t, 12H, $CH_3$ on 4 ethyls), 1.39,1.40,1.42,1.43 (4 singlets, total of 12H, $CH_3$ for four isomers, i.e., 2 cis-trans pairs), 1.75 (m, 8H, 4 $CH_2$ groups).

| combustion analysis: | calcd. for $C_9H_{18}N_2S \cdot 0.1(H_2O)$ | obs. |
|---|---|---|
| C | 57.46 | 57.90 |
| H | 9.75 | 9.24 |
| N | 14.89 | 14.94 |
| S | 17.05 | 16.68 |

The thione can be purified by column chromatography on silica gel using hexane to elute an odorous fraction before the thione. The effect of variation of temperature and concentration of the reactants on yield and reaction rate was examined in a sealed NMR tube using $D_2O$ as a solvent and sodium tosylate as an internal standard. What was observed was a smooth decrease in concentration of starting materials and smooth increase in concentration of product. The reaction time can be reduced from 16 hours at 50°C to 6 hours at 80°C. The compound, 2-methyl-2-aminobutyrothioamide, was not observed indicating that this alicyclic intermediate reacts with MEK in a fast step to yield the cyclic product.

Preparation of 2,5-diethyl-2,5-dimethylimidazolidin-4-one

A 5 liter 4-necked round-bottomed flask equipped with a mechanical stirrer and thermocouple thermometer was charged with 250 ml water and 43.3 g (0.232 mol) of 2,5-diethyl-2,5-dimethylimidazolidin-4-thione. In order to make the thione dissolve, 1.8 g NaOH was added. The solution was cooled to 0-2°C with a dry ice-acetone bath. The flask was fitted with two additional funnels. Simultaneously, a solution of 16.7 g NaOH in 100 ml water (total of 18.5 g or 0.464 mol of NaOH used) was added through one of the funnels and 105 ml (0.928 mol) of 30% $H_2O_2$ was added

through the other funnel. The reaction mixture was stirred rapidly and required extensive cooling during the addition. The 5 liter flask was used to provide a large surface area for efficient cooling of the exothermic reaction. The heat of reaction was 2692 Kcal/mol; 124. The addition was completed in 2 hours; the mixture was stirred an additional half hour.

At the end of this time, TLC indicated that no thione remained. Then, 27.9 g of $NAHSO_3$ (0.172 mol) was added to quench excess peroxide; this reaction is also somewhat exothermic (temperature increases from 26 to 43°C). The reaction mixture was transferred to a 2 liter round-bottomed flask and the solvent removed with a rotary evaporator (aspirator pressure) to give a white residue. The residue was extracted with 850 ml of boiling ethanol. Then, 50 ml of toluene was added to the solution and 130 ml of water/ethanol/toluene azeotrope distilled to remove any remaining water. The solution was cooled and filtered to remove a small amount (~ 1 g) of $Na_2SO_4$ and then the ethanol was removed on the rotary evaporator to give a syrup that crystallized on cooling to room temperature. The yield was 37.5 g (95%) mp 58-64°C. IR (nujol) 1705, 1659 cm-1. $^1$H NMR ($CDCl_3$ + $D_2O$) ppm (combination of equal amounts of 2 sets of cis-trans pairs) 0.95-0.98 (m, 12H, $CH_3$ on 4 ethyls), 1.27, 1.31, 1.34, 1.38 (4 s, total 12H, 4 $CH_3$), 1.50-1.70 (m, 8H, $CH_2$ on 4 ethyls). The singlets at 1.34 and 1.38 collapse to a singlet in $D_2O$ but now integrate 6H.

| combustion analysis: | calcd. for $C_9H_{18}N_2O$ | obs. |
|---|---|---|
| C | 63.49 | 63.07 |
| H | 10.66 | 10.01 |
| N | 16.45 | 16.23 |
| O | 9.40 | 9.49 |

Preparation of 2,5-diethyl-2,5-dimethylimidazolidin-4-one-1-oxyl

A one liter polymer jar equipped with a mechanical stirrer, bleach filled odor trap, heating mantel, reflux condenser, and thermocouple thermometer was charged with 153.3 g (0.45 mol) 20% ammonium sulfide solution. To this solution was added 1.47 g (0.03 mol) NaCN to react with the ammonium polysulfide impurity in the ammonium sulfide solution. Then, 35.9 g AN-67 (0.3 mol) and 21.7 g 2-butanone (0.3 mol) was added. The solution was stirred and heated under nitrogen at 55°C for 18 hours; some ammonia was evolved. Two liquid layers form; the lower layer is thioamide. The volume of the reaction mixture is now 200 ml. The mixture was cooled to room temperature and a solution of 36 g NaOH (0.9 mol) in 100 ml water was added. The solution was cooled to 0°C and 306 g (2.7 mol) 30% $H_2O_2$ added dropwise with stirring and cooling at 4-10°C. The addition took 65 minutes. After stirring for one hour, the solution was brought to pH = 7 by addition of a solution of 58 g concentrated $H_2SO_4$ and 56 g of water at 13°C. Then, 68 g (0.6 mol) of 30% $H_2O_2$ was added. No exotherm was noted at this time. To this was added 5.0 g $Na_2WO_4.2H_2O$. The total reaction volume is 763 ml. An initial greenish-yellow color (pertungstate ion) is replaced by a deeper yellow color (nitroxide). The temperature of the mixture climbs from 13 to 31°C over 3-1/2 hours The next day, the solution was filtered to give 27.5 g (40% yield) of nitroxide, mp 117-122°C. It was subsequently found that 1/3 of the AN-67 is destroyed by hydrolysis and irreversible reaction with sulfur to form thiocyanate ion. If this is taken into account, the yield is 82%; each step is about 93%. The solubility in NMP is at least 1:1. IR (nujol) 1720, 1675 cm-1; (toluene solution) 1713.3 cm-1. The nitroxide displays a triplet in the ESR. Aliquots of reaction mixture were withdrawn at selected times and diluted with a known amount of xylene. The integrated intensity of the ESR triplet was plotted as a function of time when the concentration of peroxide was doubled or the concentration of tungstate ion was increased to three. The data were fitted to parabolas. The initial slopes of the lines are obtained by differentiating the empirically fitted curves to determine the slope of the line and solving the equation so obtained at x = 0; the rate law at 24°C was found to be $K = k[H_2O_2]^{0.5+.1}[WO_4]^{1.0+.1}$

| combustion analysis: | calcd. for $C_9H_{17}N_2O_2 \cdot 0.03(CH_2Cl_2)$ | obs. |
|---|---|---|
| C | 57.75 | 57.71 |
| H | 9.16 | 8.82 |
| N | 14.92 | 15.21 |
| O | 17.04 | 17.28 |

**Example 2**

**Preparation** of **2,5-bis(spirocyclohexyl)imidazolidin-4-one-1-oxyl (NO-88)**

Preparation of 2,5-bis(spirocyclohexyl)imidazolidin-4-thione.

A 2 liter 4-necked round-bottomed flask equipped with a mechanical stirrer, heating mantel, reflux condenser, thermocouple therinometet, nitrogen bubbler and exit tube connected to a bleach filled odor trap was charged with 132.9 g (0.39 mol) of 20% ammonium sulfide solution followed by 0.5 g NaCN to decolorize the polysulfide impurity in the ammonium sulfide solution. Under a positive nitrogen flow, 16.1 g (0.3 mol) of ammonium chloride and 14.7 g (0.3 mol) of NaCN was added. The temperature of the solution dropped to 8°C. Then, 58.9 g (0.6 mol) of cyclohexanone that had previously been deoxygenated by bubbling nitrogen through it for 10 minutes was added dropwise while stirring during 25 minutes. The temperature rose to 30°C. The temperature of the solution was increased to 47°C, at which point external heating was stopped and the reaction spontaneously allowed to exotherm to 63°C. The temperature was then maintained at 55°C. After 1 hour, 1.0 g NaCN was added and a mild exotherm to 63°C followed by a return to 55°C was noted. An additional 1.0 g NaCN was added 30 minutes later; this caused only a mild exotherm to 58°C. The temperature was held at 55°C overnight. A sample of the resulting slurry was then withdrawn and divided into two parts. One part was dissolved in acetone, and checked by TLC (9:1 $CH_2Cl_2$:acetone); two species were present. The other part was filtered to give white crystals, mp 225-230°C. The precipitate was filtered in place with a filter stick to avoid handling the odorous mixture. The insoluble precipitate was thione; the other impurity was cyclohexanone, which remained in the filtrate. The thione was washed by adding 300 ml of water to the flask, stirring, and then removing the water through the filter stick. IR (nujol) 1520 cm-1.

Preparation of 2,5-bis(spirocyclohexyl)imidazolidin-4-one

To the wet crystalline residue obtained above was added in the same flask 24 g (0.6 mol) of NaOH dissolved in 300 ml of water. The crystals failed to dissolve; the crystals were finally dissolved by addition of 485 ml of methanol. The solution does not show the characteristic exotherm upon addition of 30% hydrogen peroxide at 0-5°C. The temperature of the solution was increased to 40°C; at this temperature addition of peroxide is exothtermic. After adding 147 g (4x0.32 mol) of 30% peroxide, the solution temperature was held at 55°C for 30 minutes and then stirred at room temperature overnight; by TLC, the mixture consisted of amide and thione. The mixture was filtered and the precipitate washed with 3x100 ml of water. To the filtrate was added 57 g of peroxide [total peroxide used = 204 g (1.8 mol)] and the solution was warmed to 40°C. A slight exotherm to 46°C occurred. To aid the oxidation, 1 g of $Na_2WO_4.2H_2O$ was added. After 15 minutes, a white precipitate began to deposit. The mixture was allowed to stir at room temperature for 18 hours and then filtered. By IR, both precipitates were identical and were combined and air dried, mp 216-220°C, 58.5 g (88% yield based on cyclohexanone). IR (nujol) 1690 cm-1.

Preparation of 2,5-bis(spirocyclohexyl)imidazolidin-4-one-1-oxyl

An acetone solution of 260 ml of 0.08M dimethyldioxirane (0.0208 mol), prepared as above, was treated with 2.3 g of 2,5-bis(spirocyclohexyl)imidazolidin-4-one dissolved in 75 ml of chloroform (previously freed of ethanol preservative by washing with 2x20 ml water and drying over magnesium sulfate) and allowed to react at room temperature overnight. The preparation was repeated using 1.7 g of amide and 270 ml of 0.0721 M dimethyldioxirane solution. Removal of the ethanol preservative is necessary to prevent the ethanol from being oxidized to acetaldehyde by the dimethyldioxirane/nitroxide system. The solvent was removed on the rotary evaporator, the residue (4.2 g) dissolved in 400 ml hot benzene, filtered to remove a trace of insoluble material, the filtrate reduced to 100 ml, rewarmed to bring all the crystals into solution, and allowed to crystallize overnight at room temperature. The yellow crystals were collected by filtration and dried in an oven at 75°C for 10 hours to give 2.4 g of nitroxide, mp 178-183°C. IR (nujol) 1707 cm-1.

| combustion analysis: | calcd. for $C_{13}H_{21}N_2O_2$ | obs. |
|---|---|---|
| C | 65.79 | 65.67 |
| H | 8.92 | 8.84 |
| N | 11.80 | 11.71 |
| O | 13.48 | 13.31 |

**Example 3-6**

**Synthesis of N-substituted imidatotidinone nitroxides**

The following Examples illustrate the process for synthesis of novel nitroxides (1, X=Alkyl).

The novel N-substituted imidazolidinone nitroxides were prepared according to the following general procedure.

A suspension of imidazolidinone nitroxide (5.5 mmol NO-67 or 1.69 mmol NO-88) and sodium hydride (1.33 molar eq., 80% dispersion in oil) was allowed to stir under an atmosphere of nitrogen in acetonitrile solvent (20 ml for NO-67 or 10 ml for NO-88) at room temperature for 15 minutes, and then added the required amount of an alkyl halide (1.20 molar equivalents). After workup and purification by column chromatography, the corresponding new N-substituted imidazolidinone nitroxide was generally obtained in good to excellent yield (45-93%)

**Example 3**

**Preparation of 2,5-Bis(spirocyclohexyl)-3-methylimidazolidin-4-one-1-oxyl (NO-88-Me)**

The title compound, NO-88-Me was isolated as a yellow solid after column chromatography (Kieselgel-60, 70-230 mesh, ethyl acetate/n-hexane 1:4 as eluent) (89.7% yield). Melting point, 103-105°C. MS (CI): 252 (M+1, 100%), 251 ($M^+$, 30.7), 237 (87.6), 236 (12.3), 235 (29.3), 222 (24.5), 221 (40.6), 196 (18.7), 193 (10.5), 142 (24.7), 140 (53.9), 112 (16.0) and 99 (23.5).

**Example 4**

**Preparation of 2,5-Diethyl-2,3,5-trimethylimidaxolidin-4-one-1-oxyl (NO-67. Me)**

The title compound, NO-67-Me was isolated (45.6% yield) after column chromatography (Kieselgel-60, 70-230 mesh, ethyl acetate/n-hexane 1:3 as eluent) as a yellow liquid. MS (CI): 200 (M+1,43.0%), 199 ($M^+$, 16.0), 186 (23.5), 185(40.0), 171 (58.0), 170 (34.0), 155 (23.0), 149 (20.2), 141 (11.6), 140 (15.6), 128 (10.0), 126(16.1), 116 (13.7), 112 (14.4), 111 (12.7), 100 (12.2), 73 (52.0).

**Example 5**

**Preparation of 2,5-Diethyl-2,5-dimethyl-3-benzylimidazolidin-4-one-1-oxyl (NO-67-Bn)**

The title compound, NO-67-Bn was isolated (93.0% yield) after column chromatography (Kieselgel-60, 70-230 mesh, ethyl acetate/n-hexane 1:5 as eluent) as a yellow solid. Melting point, 64-65°C. MS (CI): 276 (M+1, 56.7%), 275 ($M^+$, 22.0), 262 (22.0) 261 (100.0), 247(62.6), 245 (M- NO, 26.0), 231(86.0), 218 (5.3), 190 (12.3), 170 (15.0), 162 (24.6), 126 (20.1), 102 (4.0), 91 (21.5) and 72 (4.0).

**Example 6**

**Preparation of 2,5-Diethyl-2,5-dimethyl-3-a-butylimidazolidin-4-one-1-oxyl (NO-67-nBu)**

The title compound, NO-67-nBu was isolated (87.0% yield) after column chromatography (Kieselgel-60, 70-230 mesh, ethyl acetate/n-hexane 1:9 as eluent) as a yellow liquid. MS (CI): 242 (M+1, 74.0%), 241 ($M^+$, 38.3), 227 (100.0), 213 (86.7), 211 (45.0), 198 (12.0), 197 (88.0), 184 (M - nBu, 7.6), 170 (27.0), 156 (16.3), 128 (27.0), 126 (30.0), 116 (4.3), 98 (8.0) and 72 (7.6).

**Example 7**

**Preparation of 1-(2-*tert*-butoxy-1-phenylethoxy)-2,5-bis(spirocyclobexyl)-3-methylimidazolidin-4-one**

The title alkoxyamine, 1-(2-*tert*-butoxy-1-phenylethoxy)-2,5-bis(spirocyclohexy)-3-methylimidazolidin-4-one was prepared by treating 1-(2-*tert*-butoxy-1-phenylethoxy)-2,5-bis(spirocyclohexyl)-imidazolidin-4-one alkoxyamine (m.p. 244-247°C, obtained from the reaction of di-tert-butyl peroxyoxalate, styrene and nitroxide NO-88) with excess methyl iodide in the presence of sodium hydride in dimethyl sulfoxide solvent (Scheme 2). The product was isolated as a white solid in 93% yield, m.p. 129-131°C (aq. MeOH). The alkoxyamine product has improved solubility versus

its non-methylated alkoxyamine and is readily soluble in common organic solvents such as ethyl acetate, chloroform, acetone, hot methanol. $^1$H-NMR ($CDCl_3$ $\delta$ (ppm) 0.40-2.60 (m, 20H, cyclohexyl-$CH_2$), 1.10 (s, 9H, *tert*-butyl-$CH_3$), 2.90 (s, 3H, N-$CH_3$), 3.30 (dd, 1H, $(CH_3)_3COCH$), 3.66 (dd, 1H, $(CH_3)_3COCH$, 4.69 (dd, 1H, CH(Ph)ON) and 7.25 (br s, 5H, phenyl-H).

**Scheme 2**

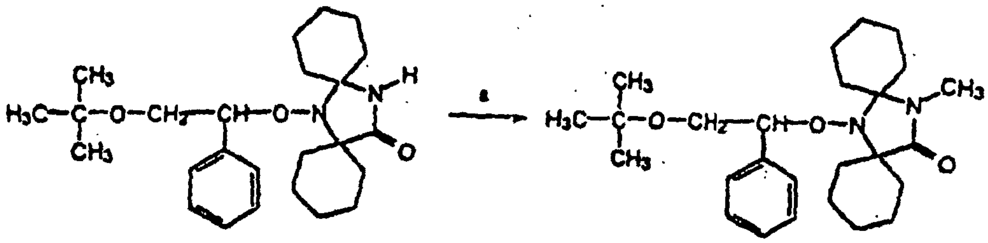


a) NaH / DMSO, excess methyl iodide, at room temperature.

## Claims

1. A colorless aqueous ammonium sulfide solution containing sodium thiocyanate and substantially no ammonium polysulfide.

2. A process for preparing the solution of claim 1 by titrating an aqueous ammonium sulfide solution containing ammonium polysulfide with sodium cyanide under nitrogen.

3. A process for making nitroxides of Formula (1):

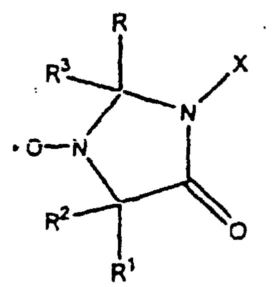

Formula (1)

wherein:

R, $R^1$, $R^2$, $R^3$ are each independently selected from the group consisting of $C_1$ to $C_{18}$ alkyl, substituted $C_1$ to $C_{18}$ alkyl, $C_6$ to $C_{18}$ aryl, $C_6$ to $C_{18}$ substituted aryl; R groups that are in a germinal position with respect to each other can together form a 4-8 membered ring; R groups that are in a cis position with respect to each other can together form a 4-8 membered ring; and

X is selected from the group consisting of hydrogen, $C_1$ to $C_{18}$ alkyl, substituted $C_1$ to $C_{18}$ alkyl, $C_6$ to $C_{18}$ aryl, $C_6$ to $C_{18}$ substituted aryl; acyl; X and R can form a 5-8 membered ring; X and $R^3$ can form a 5-8 membered ring, comprising the steps (i) preparing a colorless aqueous ammonium sulfide sotution containing sodium thiocyanate by titrating an aqueous ammonium sulfide solutions contaimng ammonium polysulfide with sodium cyanide under nitrogen; (ii) sequentially adding an aminonitrile and a ketone to the aqueous ammonium sulfide solution under nitrogen; (iii) adding base and then neutralizing; and (iv) oxidizing the reaction product of step (iii) to form the nitroxide.

4. The process of claim 3, **characterized in that** in process step (ii) aminonitrile is replaced by a mixture of kekone, ammonium chloride, and sodium or potassium cyanide.

5. The process of claim 3 or 4, **characterized in that** step (ii) is performed at a temperature of between 20° and 80°C, preferably between 30° and 60°C, and most preferably at 54°C.

6. The process of any one of claims 3 to 5, **characterized in that** the base is sodium carbonate or sodium hydroxide, most preferably sodium hydroxide.

7. The process of any one of claims 3 to 6, **characterized in that** the acid is sulfuric acid.

8. The process of any one of claims 3 to 7, **characterized in that** oxidants for step (IV) are $H_2O_2$/ tungstate, dimethyldioxirane. $H_2O_2$/acetic acid.

**Patentansprüche**

1. Farblose wässrige Ammoniumsulfidlösung, enthaltend Natriumthiocyanat und im wesentlichen kein Ammoniumpolysulfid.

2. Verfahren zur Herstellung der Lösung nach Anspruch 1 durch Titrieren einer wässrigen Ammoniumsulfidlösung, enthaltend Ammoniumpolysulfid, mit Natriumcyanid unter Stickstoff.

3. Verfahren zur Herstellung von Nitroxiden der Formel (1):

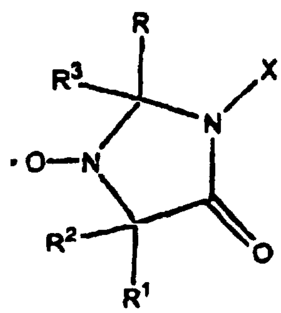

Formel (1)

wobei:

R, $R^1$, $R^2$, $R^3$ jeweils unabhängig aus der Gruppe, bestehend aus $C_1$- bis $C_{18}$-Alkyl, substituiertem $C_1$- bis $C_{18}$-Alkyl, $C_6$- bis $C_{18}$-Aryl, substituiertem $C_6$- bis $C_{18}$-Aryl, ausgewählt sind; R-Gruppen, die in einer geminalen Position in bezug zueinander sind, zusammen einen 4-8-gliedrigen Ring bilden können; R-Gruppen, die in einer cis-Position in bezug zueinander sind, zusammen einen 4-8-gliedrigen Ring bilden können; und

X aus der Gruppe, bestehend aus Wasserstoff, $C_1$- bis $C_{18}$-Alkyl, substituiertem $C_1$- bis $C_{18}$-Alkyl, $C_6$- bis $C_{18}$-Aryl, substituiertem $C_6$- bis $C_{18}$-Aryl; Acyl, ausgewählt ist; X und R einen 5-8-gliedrigen Ring bilden können; X und $R^1$ einen 5-8-gliedrigen Ring bilden können,

umfassend die Schritte

(i) Herstellen einer farblosen wässrigen Ammoniumsulfidlösung, enthaltend Natriumthiocyanat, durch Titrieren einer wässrigen Ammoniumsulfidlösung, enthaltend Ammoniumpolysulfid, mit Natriumcyanid unter Stickstoff; (ii) aufeinanderfolgend Hinzufügen eines Aminonitrils und eines Ketons zu der wässrigen Ammoniumsulfidlösung unter Stickstoff; (iii) Hinzufügen von Base und dann Neutralisieren; und (iv) Oxidieren des Reaktionsprodukts von Schritt (iii), um das Nitroxid zu erzeugen.

**4.** Verfahren nach Anspruch 3, **dadurch gekennzeichnet, daß** in Verfahrensschritt (ii) Aminonitril durch ein Gemisch von Keton, Ammoniumchlorid und Natrium- oder Kaliumcyanid ersetzt wird.

**5.** Verfahren nach Anspruch 3 oder 4, **dadurch gekennzeichnet, daß** Schritt (ii) bei einer Temperatur zwischen 20° und 80°C, vorzugsweise zwischen 30° und 60°C und am meisten bevorzugt bei 54°C, durchgerührt wird.

**6.** Verfahren nach einem der Ansprüche 3 bis 5, **dadurch gekennzeichnet, daß** die Base Natriumcarbonat oder Natriumhydroxid, am meisten bevorzugt Natriumhydroxid, ist.

**7.** Verfahren nach einem der Ansprüche 3 bis 6, **dadurch gekennzeichnet, daß** die Säure Schwefelsäure ist.

**8.** Verfahren nach einem der Ansprüche 3 bis 7, **dadurch gekennzeichnet, daß** Oxidationsmittel für Schritt (iv) $H_2O_2$/Wolframat, Dimethyldioxiran, $H_2O_2$/Essigsäure sind.

**Revendications**

**1.** Solution aqueuse incolore de sulfure d'ammonium contenant du thiocyanate de sodium et substantiellement pas de polysulfure d'ammonium.

**2.** Procédé pour préparer la solution selon la revendication 1 par titrage d'une solution aqueuse de sulfure d'ammonium contenant du polysulfure d'ammonium avec du cyanure de sodium sous azote.

**3.** Procédé de fabrication de nitroxydes de formule (1) :

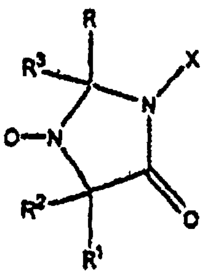

Formule (I)

dans laquelle
R, $R^1$, $R^2$, $R^3$ sont chacun indépendamment choisis parmi le groupe constitué d'alkyles en $C_1$-$C_{18}$, d'alkyles en $C_1$-$C_{18}$ substitués, d'aryles en $C_6$-$C_{18}$, d'aryles en $C_6$-$C_{18}$ substitués ; les groupements R qui sont dans une position jumelle l'un par rapport à l'autre peuvent conjointement former un cycle à 4-8 chaînons ; les groupements R qui sont en position cis l'un par rapport à l'autre peuvent conjointement former un cycle à 4-8 chaînons ; et
X est choisi parmi le groupe constitué d'hydrogène, d'alkyles en $C_1$-$C_{18}$, d'alkyles en $C_1$-$C_{18}$ substitués, d'aryles en $C_6$-$C_{18}$, d'aryles en $C_6$-$C_{18}$ substitués, d'acyles ; X et R peuvent former un cycle à 5-8 chaînons ; X et $R^3$ peuvent former un cycle à 5-8 chaînons, comprenant les étapes de (i) préparation d'une solution aqueuse incolore de sulfure d'ammonium contenant du thiocyanate de sodium par titrage d'une solution aqueuse de sulfure d'ammonium contenant du polysulfure d'ammonium avec du cyanure de sodium sous azote ; (ii) addition séquentielle d'un aminonitrile et d'une cétone à la solution aqueuse de sulfure d'ammonium sous azote ; (iii) addition de base et ensuite neutralisation ; et (iv) oxydation du produit de la réaction de l'étape (iii) pour former le nitroxyde.

**4.** Procédé selon la revendication 3 **caractérisé en ce que** dans l'étape (ii) du processus, l'aminonitrile est remplacé par un mélange de cétone, de chlorure d'ammonium et de cyanure de sodium ou de potassium.

**5.** Procédé selon la revendication 3 ou 4, **caractérisé en ce que** l'étape (ii) est effectuée à une température entre 20 et 80°C, de préférence entre 30 et 60°C, et davantage préféré à 54°C.

**6.** Procédé selon l'une quelconque des revendications 3 à 5, **caractérisé en ce que** la base est du carbonate de

sodium ou de l'hydroxyde de sodium, davantage préféré de l'hydroxyde de sodium.

7. Procédé selon l'une quelconque des revendications 3 à 6, **caractérisé en ce que** l'acide est l'acide sulfurique.

8. Procédé selon l'une quelconque des revendications 3 à 7, **caractérisé en ce que** les oxydants pour l'étape (iv) sont un mélange $H_2O_2$/tungstate, le diméthyldioxirane, un mélange $H_2O_2$/acide acétique.